# EUROPEAN PATENT SPECIFICATION

(11) **EP 2 788 765 B1**
(45) Date of publication and mention of the grant of the patent: **15.02.2017**
(21) Application number: 12797927.6
(22) Date of filing: 07.12.2012
(51) Int. Cl.: G01N 33/569, C12Q 1/06

(54) **METHOD FOR DETECTION OF BACTERIA IN MILK**
VERFAHREN ZUM NACHWEIS VON BAKTERIEN IN MILCH
PROCÉDÉ DE DÉTECTION DE BACTÉRIES DANS LE LAIT

(30) Priority: 09.12.2011 EP 11192838
(43) Date of publication of application: 15.10.2014
(73) Proprietor: Sietzema, Sietze, 8385 GS Vledderveen (NL)
(72) Inventor: Sietzema, Sietze, 8385 GS Vledderveen (NL)
(74) Representative: Maiwald Patentanwalts GmbH
(86) International application number: PCT/EP2012/074742
(87) International publication number: WO 2013/083754

(56) References cited:
- WO-A1-02/075310
- KR-A- 20010 028 258
- RUEGG PAMELA L ET AL: "Milk quality and mastitis tests", BOVINE PRACTITIONER, STILLWATER, OK, US, vol. 36, no. 1, 1 February 2002 (2002-02-01), pages 41-54, XP009167124, ISSN: 0524-1685
- R G Mcclelland ET AL: "Detection of Salmonella typhimunrum in Dairy Products with Flow Cytometry and Monoclonal Antibodies", APPLIED AND ENVIRONMENTAL MICROBIOLOGY, 1 December 1994 (1994-12-01), pages 4255-4262, XP055195977, Retrieved from the Internet: URL:http://www.ncbi.nlm.nih.gov/pmc/articl es/PMC201977/pdf/aem00029-0049.pdf [retrieved on 2015-06-16]
- Liju Yang ET AL: "Simultaneous detection of Escherichia coli O157:H7 and Salmonella Typhimurium using quantum dots as fluorescence labels", The Analyst, vol. 131, no. 3, 1 January 2006 (2006-01-01), pages 394-401, XP055237379, GB ISSN: 0003-2654, DOI: 10.1039/B510888H
- BAUMGARTH<1> N ET AL: "A practical approach to multicolor flow cytometry for immunophenotyping", JOURNAL OF IMMUNOLOGICAL METHODS, ELSEVIER SCIENCE PUBLISHERS B.V.,AMSTERDAM, NL, vol. 243, no. 1-2, 21 September 2000 (2000-09-21), pages 77-97, XP004210694, ISSN: 0022-1759, DOI: 10.1016/S0022-1759(00)00229-5
- Nobuyasu Yamaguchi ET AL: "Rapid detection of respiringEscherichia coli O157:H7 in apple juice, milk, and ground beef by flow cytometry", Cytometry, vol. 54A, no. 1, 16 June 2003 (2003-06-16) , pages 27-35, XP055007975, ISSN: 0196-4763, DOI: 10.1002/cyto.a.10045

## Description

### Background of the Invention

Certain bacteria are causing illness, especially mastitis, in milk cows which illness may negatively affect the quality of the milk. E. g., mastitis is an infectious decease in the udder or nipples of an individual cow, and mastitis bacteria will generally be present in the milk produced from such cows. Various different types of bacteria may be involved in causing mastitis, i.e. there is not just one type or strain of mastitis bacterium, but many. The types of bacteria involved in mastitis generally vary from case to case.

Since milk products are generally, especially in industrial size dairy operations, produced by mixing the milk from a multiplicity of cows, said products will contain a certain quantity of undesirable germs, e.g. mastitis-causing bacteria, when some cows are infected, even if the majority of the cows generating the milk are healthy. However, there is no a priori predictability of the types and concentrations of the bacteria cells, comprised by infected milk which ends up being mixed into the finally collected milk.

Mastitis is controlled by the government in milk samples for payment control. Too high a concentration of bacteria in the delivered milk is an indication of mastitis, such that the milk producer obtains only a lower price per quantity of delivered milk. After several deliveries with high bacterial cell count, milk delivery may actually even be forbidden.

When mastitis occurs, the veterinarians (or the milk producers) are often using a cocktail of antibiotics in order to kill and/or dismantle the bacteria, in the hope that the cocktail is covering the actually present bacterial spectrum, but without initially determining which types of bacteria are indeed involved. In the worst case, wrong antibiotics, or antibiotics not specifically suited for that particular type or types of bacteria are used one after another, or simultaneously.

This uncontrolled use of antibiotics is causing more and more bacteria mutations which show increased immunity against antibiotics. This is a dangerous situation also for public health.

### Summary of the Invention

It is an important objective of the invention to create an improved method for detecting bacteria or different types of bacteria in milk, especially in a (fully or at least partly) automatic detection system. In preferred embodiments, the invention permits the selective and specific detection of bacteria or types of bacteria, and prominently among these, mastitis-causing bacteria. The invention enables such specific detection also where more than one type of bacterium is present in the milk, since it enables the detection of several different types of bacteria side by side, or simultaneously. The inventive method further makes it possible to determine the type or types of bacteria that is/are causing the infection, e. g. mastitis, in order to be able to select and dose the right antibiotics to the cow.
These and other objectives are attained by the subject matter defined in the attached claims.

More specifically, an improved method for detecting bacteria in milk incorporates the steps defined in claim 1. The milk is contacted with a preparation comprising an effective amount of more than one type of antibodies, each type binding to a different type of bacteria to be detected; or a plurality of such types;
said antibodies are stained, before or after said contacting, with a staining preparation, said staining preparation being selected so that the antibodies stained therewith exhibit a difference in color concentration and/or change in color when in contact with said bacteria; the concentration of stained antibodies bound to bacteria in said milk is determined from the presence and, in case, the relative intensity of the color caused by contacting said milk with said stained antibodies without separating the bacteria from the milk, by using a fluorescent antibody stain and a flow cytometer as the detector. These steps may be used in any suitable sequence, wherein generally, however, the step utilizing the color concentration produced by combining the stained antibodies with the bacteria will necessarily follow the step of contacting said antibodies and said bacteria.
In preferred embodiments, the inventive method involves:
Said method, wherein said antibodies are capable of specifically binding to bacteria involved in mastitis;
Said method, wherein said preparation comprises more than two, more preferably more than three, even more preferably more than four and most preferably five or more different types of antibodies, each type specifically binding to a different type of bacterium or a plurality of such types.

The color change and/or concentration change can be used to determine the type of cells, especially bacteria, in the milk, and preferably also to determine the relative amount of said cells.

It is generally known to detect bacteria by using specific antibodies in body tissue and fluids in the medical art. Specific conditions apply however to such known uses, which set such known methods apart from the inventive method.

### Detailed Description

Mastitis results in an increased amount of bacteria per weight or volume unit of milk. The bacteria may be counted by the flow cytometer principle by use of fluorescence technique.
Fluorescence technique means in this case that the bacteria are combined (and thus, reacted) with a suitably stained antibody and the combination is passed through a flow cell where under the influence of fluorescence light, the individual cells combined with the antibodies give a light pulse. The number of light pulses is detected and is in correlation with the number of bacterial cells.

There are several groups of bacteria causing mastitis. The invention is based on the fact that specific antibodies only bind to specific types of bacteria. With this data it is possible to identify different bacteria by the use of different antibodies.
It is possible to use a single type of antibody, to detect and measure the concentration of basically a single type of bacterium. However, since there will generally be more than one type of bacterium involved, and there often is no a priori information on which kinds of bacteria will be encountered, it is preferred to add up to 5 (or even more) different sorts of antibodies into a milk sample. Preferably, these will be selected to encompass the most common types of bacteria encountered in practice. Each group of antibodies previously are stained with a corresponding number (up to 5 or more) specific different staining solutions. E. g., using 5 different stainings makes it possible that 5 different colors are present and these can be individually detected by specific Method 1 A.

### Method 1 A

When stained antibodies are surrounding (attached to, or binding to) bacteria in a milk sample, the color of the milk (or cleared milk) is changing under the influence of the bacteria. This change of color can be detected by a specific color detector.

When the bacteria are passing through a flow cell provided with one or more different color detectors (fluorescence light), the number of bacteria can be counted based on the color change caused by the stained antibodies.
This color change is depending on the type of stained antibodies. The used antibodies may be particularly selected for that particular type of bacterium, if that is already known. It is generally known which type or types of bacteria are causing mastitis, and the choice of stained antibodies will generally be based on this knowledge. By this way it can be detected and measured which type of bacterium is causing a disease in the cow, e.g. mastitis.

### Method 1 B

Stained antibodies that bind to specific unstained bacteria in a milk sample will under influence of fluorescence light in a flow cytometer cause a light pulse. Because of the fact that several antibodies will bind to a single bacterium, there is a difference in light output pulses between a single antibody free in the milk sample and multiplicities of antibodies around the bacteria passing through the flow cytometer and thus, the bacteria can be detected by the different light pulse intensity produced.

### Method 1 C

Using the principle of antibodies bound to bacteria, as shown in Method 1 A and Method 1 B, the detection can also be carried out by filtering the milk sample. To detect the bacteria in the milk sample surrounded (attached or bound) to antibodies, the milk sample can be micro-filtered in such a way that all bacteria, surrounded or not surrounded by antibodies, will stay on the filter surface. On the filter surface, a microscope or any other detection system, fluorescence or non-fluorescence, can be used to identify or recognize the antibodies-surrounded bacteria.

The flow cytometer preferably has 5 or more different color detectors. These detectors are reacting to 5 different colors. When a flow of stained bacteria passes the microscope objective, there will be a number of pulses corresponding with the number of bacteria passing.

When between the stained bacteria, also pulses (approx. same size) with different colors are detected, this is an indication of antibody reaction on a mastitis caused by different bacteria and with that the choice of antibiotics can be made.

## Claims

1. A method of detecting the presence of bacteria in milk, wherein milk is contacted with a preparation comprising an effective amount of at least one type of antibodies, said antibodies being capable of specifically binding to bacteria to be detected;
staining said antibodies, before or after said contacting, with a staining preparation, said staining preparation being selected so that the antibodies stained therewith exhibit a color change or a change in color concentration when in contact with said bacteria; and
determining the concentration of stained antibodies bound to bacteria in said milk from the presence and/or the relative intensity of the color change caused by contacting said milk with said stained antibodies, **characterized in that** said preparation comprises more than one type of antibodies, each antibody type specifically binding to a different type of bacteria or to a plurality of such bacteria types, wherein the color change is detected, qualitatively and/or quantitatively, without separating the bacteria from the milk, by using a fluorescent antibody stain and a flow cytometer as the detector.

2. The method of claim 1, wherein said antibodies are capable of specifically binding to bacteria involved in mastitis.

3. The method of claim 2, wherein said preparation comprises more than two, more preferably more than three, even more preferably more than four and most preferably five or more different types of antibodies, each type specifically binding to a different type of bacteria or a plurality of such types.

4. The method of any one of the preceding claims, wherein the color change is used to determine the type or types of bacteria in the milk, and preferably also to determine the relative amount (concentration) of said bacteria.

## Patentansprüche

1. Ein Verfahren zum Nachweis des Vorliegens von Bakterien in Milch, wobei die Milch mit einer Zubereitung kontaktiert wird, welche eine wirksame Menge mindestens eines Typs von Antikörpern umfasst, wobei die Antikörper in der Lage sind spezifisch an die nachzuweisenden Bakterien zu binden;
Färben der Antikörper, vor oder nach dem Kontaktieren, mit einer Färbungszubereitung, wobei die Färbungszubereitung so ausgewählt ist, dass die damit gefärbten Antikörper eine Farbveränderung oder eine Veränderung der Farbkonzentration aufweisen, wenn sie in Kontakt mit den Bakterien kommen; und
Bestimmen der Konzentration der gefärbten Antikörper gebunden an Bakterien in der Milch aus der Gegenwart und/oder der relativen Intensität der Farbveränderung, welche durch das Kontaktieren der Milch mit den gefärbten Antikörpern verursacht wird,
**dadurch gekennzeichnet, dass** die Zubereitung mehr als einen Antikörpertyp umfasst, wobei jeder Antikörpertyp spezifisch an einen unterschiedlichen Bakterientyp bindet oder an eine Vielzahl von derartigen Bakterientypen, wobei die Farbveränderung detektiert wird, qualitativ und/oder quantitativ, ohne die Bakterien von der Milch zu separieren, durch Verwendung eines fluoreszenten Antikörperfarbstoffs und eines Durchflusszytometers als Detektor.

2. Verfahren nach Anspruch 1, wobei die Antikörper in der Lage sind an Bakterien involviert in Mastitis zu binden.

3. Verfahren nach Anspruch 2, wobei die Zubereitung mehr als zwei, vorzugsweise mehr als drei, besonders bevorzugt mehr als vier und insbesondere bevorzugt fünf oder mehr verschiedene Arten von Antikörpern umfasst, wobei jeder Typ spezifisch an einen unterschiedlichen Bakterientypen oder einer Vielzahl derartiger Typen bindet.

4. Verfahren nach einem der vorhergehenden Ansprüche, wobei die Farbveränderung verwendet wird, um den Typ oder die Typen der Bakterien in der Milch zu bestimmen, und vorzugsweise auch um die relative Menge (Konzentration) des Bakteriums zu bestimmen.

## Revendications

1. Un procédé de détection de la présence de bactéries dans le lait, dans lequel le lait est mis en contact avec une préparation comprenant une quantité efficace d'au moins un type d'anticorps, lesdits anticorps étant capables de se lier spécifiquement aux bactéries devant être détectées ;
de coloration desdits anticorps, avant ou après ladite mise en contact, à l'aide d'une préparation colorante, ladite préparation colorante étant sélectionnée de sorte que les anticorps qu'elle colore présentent un changement de couleur ou un changement de concentration de couleur une fois en contact avec lesdites bactéries ; et
de détermination de la concentration des anticorps colorés liés à la bactérie dans ledit lait à partir de la présence et/ou l'intensité relative du changement de couleur causé par la mise en contact dudit lait avec lesdits anticorps colorés, **caractérisé en ce que** ladite préparation comprend plus d'un type d'anticorps, chaque type d'anticorps se liant spécifiquement à un type différent de bactéries ou à une pluralité de tels types de bactéries, dans lequel le changement de couleur est détecté, qualitativement et/ou quantitativement, sans séparer les bactéries du lait, à l'aide d'une coloration fluorescente d'anticorps et d'un cytomètre de flux comme détecteur.

2. Le procédé selon la revendication 1, dans lequel lesdits anticorps sont capables de se lier à des bactéries impliquées dans la mastite.

3. Le procédé selon la revendication 2, dans lequel ladite préparation comprend plus de deux, de préférence plus de trois, encore plus préférentiellement plus de quatre et encore plus préférentiellement plus de cinq ou davantage types différents d'anticorps, chaque type se liant spécifiquement à un type différent de bactéries ou à une pluralité de tels types.

4. Le procédé selon l'une quelconque des revendications précédentes, dans lequel le changement de couleur permet de déterminer le ou les type(s) de bactéries dans le lait, et de préférence permet également de déterminer la quantité relative (concentration) desdites bactéries.
